# EUROPEAN PATENT SPECIFICATION

(11) **EP 3 678 498 B1**
(45) Date of publication and mention of the grant of the patent: **10.11.2021**
(21) Application number: 18778810.4
(22) Date of filing: 06.09.2018
(51) Int. Cl.: A23L 33/10, A23L 33/16, A61K 31/194, A61K 31/7004, A61P 3/14

(54) **COMPOSITION FOR CALCIUM SUPPLEMENTATION**
ZUSAMMENSETZUNG ZUR KALZIUMERGÄNZUNG
COMPOSITION POUR SUPPLÉMENTATION DE CALCIUM

(30) Priority: 06.09.2017 IT 201700099708
(43) Date of publication of application: 15.07.2020
(73) Proprietor: Abiogen Pharma S.p.A., 56121 Pisa (IT)
(72) Inventor: DINI, Laura, 56127 Pisa (IT); NEGGIANI, Fabio, 56123 Pisa (IT); CHETONI, Patrizia, 56025 Pontedera (IT); TAMPUCCI, Silvia, 56124 Pisa (IT)
(74) Representative: Cattaneo, Elisabetta
(86) International application number: PCT/EP2018/073981
(87) International publication number: WO 2019/048534

(56) References cited:
- WO-A1-2010/090614
- US-A- 5 232 709
- US-A- 5 759 575
- US-A1- 2007 065 542
- US-A1- 2009 041 860

## Description

### FIELD OF THE INVENTION

The invention concerns an aqueous suspension formulation comprising calcium citrate that may be used for calcium supplementation in subjects in need of the above supplementation.

The use of said composition prevents and counteracts the onset of diseases related to bone mass loss, such as osteoporosis and fractures.

### STATE OF THE ART

Calcium is one of the most abundant minerals in the human organism, the major constituent of bones and teeth, and plays an important role in various physiological systems. Since our body does not produce minerals, its content is totally dependent on its external intake through diet or supplementation.

Its correct intake is therefore essential for the development of teeth in children, and for bone health throughout the entire life of a human being. The level of calcium in our body is, in fact, also one of the main factors involved in the development of osteoporosis in elderly subjects, who represent an increasingly large segment of the population, given the lengthening of the average population lifetime, especially in the more developed countries.

In 1994, for example, in the United States, the National Health Institute (NIH) Consensus Development Panel reviewed the recommended daily intake for calcium, which was established between 800 and 1500 mg per day, depending on age. Over time, numerous formulations of calcium supplements and drugs have been developed to allow the correct supplementation to meet its requirements.

The most widespread products on the international market are products based on calcium carbonate, mainly in the form of a tablet, in various dosages, mainly of 500 and 1000 mg.

While these products obviously meet the need for calcium supplementation, on the other hand they are not exempt from side effects. As it is by now well known in the literature, in fact, prolonged intake of calcium carbonate is often associated with the onset of kidney stones (nephrolithiasis).

De facto, the many pharmaceutical development attempts carried out so far by companies operating in this sector, are aimed at the research of calcium salts alternative to calcium carbonate, that are readily absorbable in the digestive tract, more bioavailable, and therefore able to provide more effectively the desired calcium doses, while inhibiting calcium nephrolithiasis.

Various calcium salts have been considered over time (phosphate, citrate, chloride, acetate, lactate, gluconolactate, etc.), each of which provides a different amount of calcium, depending on the molecular weight, and has its own solubility, that during transit through the intestinal tract responsible for absorption determines the bioavailability thereof.

One of these, calcium chloride, for example, is a salt with high solubility that is currently used exclusively by intravenous injection route in the case of cardiopulmonary emergencies; its oral intake, as a calcium supplement, especially for long periods, would not be possible, because it would cause irritation of the mucous membranes of the entire digestive tract, would put patients at risk of acidosis in the blood and urine, and would require constant monitoring of levels of carbon dioxide and chlorine in patients.

Alternatively, the use of calcium phosphate, a salt which is also insoluble and with a calcium content of 40%, fully comparable to calcium carbonate, would not have any particular advantage, neither in formulation nor in clinical terms, with respect to carbonate, being *inter alia* not usable in subjects with achlorhydria, such as the elderly, who represent the population segment most in need of supplementation, for which the clinical efficacy of this salt seems to be even lower than that of carbonate. The potential use, instead, of calcium acetate, already known and used in clinical practice by virtue of its chelating properties towards phosphate ions, which make it the drug of choice in cases of severe renal insufficiency, would not be possible in the dosages required to allow a correct supplementation of calcium, as it would magnify the typical side effects of its chronic intake, such as abdominal pain, constipation, or diarrhea.

A further salt that has been particularly studied over the years, proving to be considered a valid alternative to calcium carbonate, is calcium citrate.

This salt in fact exhibits a high bioavailability, even about double the bioavailability of calcium phosphate, and about 20-30% higher than that of calcium carbonate. This greater bioavailability seems to be dependent on the different absorption mechanisms involved at the intestinal level: calcium is absorbed at the intestinal level by active transport when in the ionized form, and by passive diffusion through the paracellular pathway when calcium is in the form of a complex with the citrate ion.

It is also perfectly tolerated, and therefore usable in subjects with achlorhydria, it is thus suitable for the wide elderly patients segment, for subjects with inflammatory bowel disease, for subjects with absorption disorders, for subjects treated with H2-blockers or proton pump inhibitors, and it is associated with very reduced risks of development of kidney stones (nephrolithiasis), compared to calcium carbonate, being the citrate ions inhibitors of crystallization.

In addition, as it can be freely taken at any time of the day, both in the presence and in the absence of food, it gives the pharmaceutical product or integrator containing it a wide flexibility of use.

Therefore, calcium citrate seems to represent nowadays the most effective therapeutic alternative, and with fewer side effects compared to calcium carbonate, if one wants to prepare pharmaceutical products or supplements for calcium supplementation.

From a formulative point of view, the use of calcium citrate is, however, far from simple for various reasons.

Firstly, calcium citrate molecule contains only about 21% of calcium (compared to, for example, 40% in the molecule of calcium carbonate), therefore, during the preparation of pharmaceutical products or tablet supplements, in order to administer equal dosages of calcium, typically 500 or 1000 mg per unitary dose, it is necessary to achieve volumes and weights for the single doses proportionally much higher, which in fact make it impossible to prepare tablets having such dosages. Such tablets would, in fact, be more fragile, brittle, difficult to machine and therefore to be produced, and, in any case, should the technological problems related to their production be overcome, very large tablets, difficult to swallow by the patients, especially by the elderly and children, would be obtained.

The few formulations, based on calcium citrate alone, that reached the market have in fact much lower dosages than those recommended, typically around 200 mg of calcium per unitary dose, and involve poor compliance, requiring patients to take 3-5 daily tablets to reach the recommended daily dose.

Nevertheless, in the attempt to administer calcium in the form of calcium citrate, formulations in the form of solubilizable powders at the time of intake (for example, powders or effervescent tablets) by dissolution in certain volumes of water, typically not less than 150-200 ml per dose, have been studied and developed over time.

These preparations typically contain calcium carbonate or other calcium salts, also in mixture thereof, and citric acid, or citric acid in mixture with other organic acids, such as for example tartaric acid or malic acid, which favor calcium solubilization in the solution and therefore allow partial or total conversion into readily assimilable soluble citrate calcium.

In reality, this type of pharmaceutical preparations still have disadvantages, in terms of patient compliance, as they require the availability of water at the time of the intake, which patients does not always have with them, and which would in any case be forced to find; the need to ingest a volume of preparation not less than 150-200 ml, that is a fair amount of liquid that not all patients like to drink; as well as the fact that the calcium salt present in the preparation takes a few minutes to completely solubilize and, still, tends to reprecipitate easily.

These evidences and limits clearly impact on the easiness and pleasantness of the preparation intake for the patient, thus reducing compliance.

As an example, in the patent document US 5,759,575, a wide review of the solution attempts investigated over time to solve the issues related to poor solubility of calcium salts, their tendency to reprecipitate, and the fact that the effervescent powders and tablets prepared are often difficult to machine, is reported.

The object of the present invention is therefore to provide a formulation comprising calcium citrate which does not give rise to the aforementioned disadvantages.

In particular, the object of the present invention is to provide a formulation comprising calcium citrate which can be easily taken in any situation, without the need to find a solution in which to perform its dissolution.

The object of the present invention is therefore to provide a formulation comprising calcium citrate which can be ready-to-use, easy to take by both elderly and pediatric patients.

Therefore, the ultimate object of the present invention is to provide a ready-to-use formulation comprising calcium citrate which can be easily taken by patients of any age and physical conditions, so as to ensure the recommended daily calcium intake, thus preventing and counteracting the onset of diseases related to bone mass loss, such as osteoporosis and fractures.

### SUMMARY OF THE INVENTION

The inventors have surprisingly found that the objects of the present invention could be achieved by making a formulation in the form of an aqueous suspension comprising calcium citrate and isomalt.

### DETAILED DESCRIPTION OF THE INVENTION

The invention therefore concerns a formulation in the form of an aqueous suspension comprising calcium citrate and isomalt.

For the purposes of the present invention, the term "calcium citrate" is intended to mean any salt form obtainable between calcium ions and citrate ions, even in hydrated form, as well as any amorphous or polymorphous form thereof.

Preferably said calcium citrate is calcium citrate tetrahydrate.

The formulation in the form of an aqueous suspension of the invention comprises calcium citrate in a concentration, expressed as calcium, in the range from 20 to 100 mg per 1 ml of suspension, more preferably in the range from 40 to 70 mg per 1 ml of suspension, still more preferably of about 50 mg per 1 ml of suspension.

Said formulation in the form of an aqueous suspension of the invention comprises isomalt in a percentage concentration (w/w) in the range from 0.5 to 10%(w/w), more preferably from 2 to 6%(w/w) with respect to the weight of the final formulation.

In a particularly preferred embodiment of the invention, the formulation in the form of an aqueous suspension of the invention further comprises sorbitol. Preferably said sorbitol is present in a percentage concentration (w/w) in the range from 10 to 30% (w/w), preferably from 17 to 28%(w/w) with respect to the weight of the final formulation.

As it will be apparent from the experimental part that follows, the inventors surprisingly discovered that by suspending calcium citrate salt in an aqueous solution in the presence of isomalt, a ready-to-use suspension, stable over time, was obtained. The invention is surprising since many types of solubilizing agents, complexing agents, viscosizing agents, and cosolvents were tested, individually and as mixture thereof, in order to obtain stable calcium citrate suspensions, but in most cases, even where an initial suspension was obtained, the same quickly tended to settle, already within a few hours, or at most a few days, giving rise to insoluble "cakes".

The specific use of isomalt, commonly known and used as a sweetening agent, once added to aqueous suspensions of calcium citrate, made surprisingly and unexpectedly possible the preparation of stable suspensions, not subject to sedimentation, with pleasant organoleptic characteristics.

The performance of these suspensions was even higher when they also included sorbitol.

The stability of these suspensions was so high also to allow the preparation of formulations containing very high doses of calcium citrate in small suspension volumes.

In fact, the aqueous suspension formulation of the invention allows to dose a concentration of calcium citrate, expressed with reference to calcium, up to 100 mg per 1 ml of suspension.

The aqueous suspension formulation of the invention can also be advantageously prepared as a unitary dose.

Said aqueous suspension of the invention in unitary dosage form comprises calcium citrate in an amount corresponding to a unitary dose of calcium in the range from 200 to 1500 mg, preferably from 400 to 1200 mg, still more preferably of 500 or 1000 mg. Said aqueous suspension of the invention in unitary dosage form comprises isomalt in an amount in the range from 0.1 to 1 g, preferably from 0.4 to 1 g.

Said aqueous suspension of the invention in unitary dosage form may further comprise sorbitol in an amount in the range from 1 to 5 g, preferably from 2.5 to 5 g. Said aqueous suspension of the invention in unitary dosage form has a total weight in the range from 5 to 20 g, preferably in the range from 6 to 10 g for the unitary dose delivering an equivalent amount of calcium of about 500 mg, and in the range from 16 to 20 g for the unitary dose delivering an equivalent amount of calcium of about 1000 mg.

Said aqueous suspension of the invention in unitary dosage form has a total volume in the range from 5 to 30 ml, preferably from 8 to 25 ml, more preferably from 9 to 15 ml, still more preferably of about 10 ml.

In particular, the aqueous suspension of the invention allows the administration of a unitary dose of 1000 mg of calcium, by the intake of a reduced volume of preparation generally comprised between 9 and 15 ml, preferably of about 10 ml, in the form of readily assimilable calcium citrate.

The possibility offered by the aqueous suspension of the present invention to provide a unitary dose of 1000 mg of calcium also allows, and by a single intake, to administer the entire daily calcium dosage recommended by the scientific and medical community.

In addition, the possibility offered by the aqueous suspension to provide the unitary dose of 1000 mg of calcium in such a small volume, makes it easy and pleasant its intake even by subjects with swallowing difficulties.

The formulation in the form of an aqueous suspension of the invention can therefore be readily taken from elderly patients and from pediatric patients.

Of course, such a formulation may also be prepared in different sizes, comprising lower dosages of calcium, such as typically the unitary dose sizes of 500 mg of calcium, 250 mg of calcium, 200 mg of calcium, or any other dosage considered useful.

The aqueous suspension formulation of the invention, also in unitary dosage form, comprising calcium citrate and isomalt, and preferably also sorbitol, may further comprise any pharmaceutical excipients useful for the preparation thereof, such as for example pH modifiers, cosolvents, solubilizers, stabilizers, preservatives, sweeteners, flavoring agents.

Among pH modifiers, for example, organic acids, preferably citric acid or malic acid, are used.

Among cosolvents, preferably glycerin can be cited.

Among solubilizers, preferably sodium citrate, sodium gluconate, sodium glucono-delta-lactone, or mixtures thereof, can be cited.

Among sweeteners, preferably sodium saccharinate or sucralose can be cited. Among preservatives, preferably methyl paraben, propyl paraben, or mixtures thereof, can be cited.

The aqueous suspension of the invention can also be easily flavored, in order to hide the typical "chalky" flavor of these preparations, so as to obtain a suspension that is more palatable and easier to take by patients, especially by pediatric patients. Typical flavors that can be used are, for example, creme caramel flavor, orange flavor, cherry flavor, strawberry flavor, lemon flavor.

Preferably the aqueous suspension of the invention therefore also comprises a flavor, more preferably said flavor is cherry flavor.

In a preferred embodiment of the invention, the invention concerns an aqueous suspension formulation comprising calcium citrate tetrahydrate, isomalt, sorbitol, citric acid, and sodium citrate.

In a still more preferred embodiment, said aqueous suspension comprises calcium citrate tetrahydrate, isomalt, sorbitol, citric acid, sodium citrate, methyl paraben, propyl paraben, and cherry flavor.

The formulation in the form of an aqueous suspension of the invention may therefore be effectively used in the calcium supplementation of any subject in need of supplementation, therefore, for example, in the prevention and treatment of diseases characterized by bone mass loss, such as osteoporosis, fractures, chronic diarrhea syndromes, hypertension, colon cancer.

Furthermore, the aqueous suspension of the invention, filled into flexible containers, such as for example cheer pack or stick pack, preferably stick pack, makes it possible for the patient the intake in any place or time of the day, since these containers are not bulky and are easily transportable, they can also be worn inside clothes, for example simply inside pockets.

The invention, therefore, also concerns a single-dose container, preferably flexible, still more preferably a stick pack, containing a pharmaceutical preparation or nutritional supplement in the form of an aqueous suspension comprising calcium citrate and isomalt, preferably sorbitol as well.

Preferably said calcium citrate is calcium citrate tetrahydrate.

More preferably said calcium citrate tetrahydrate is contained in such an amount as to provide an equivalent unitary dose of calcium of 200, 250, 500 or 1000 mg, still more preferably of 500 or 1000 mg.

Furthermore, said pharmaceutical preparation or nutritional supplement may further comprise any pharmaceutical excipients useful for the preparation thereof, such as for example pH modifiers, cosolvents, solubilizers, stabilizers, preservatives, sweeteners, flavoring agents.

Among usable excipients there are citric acid, malic acid, glycerin, sodium citrate, sodium gluconate, sodium glucono-delta-lactone, sodium saccharinate, sucralose, methyl paraben, propyl paraben, or mixtures thereof.

In a further embodiment of the invention, the invention therefore concerns a single-dose stick pack containing an aqueous suspension comprising calcium citrate, preferably calcium citrate tetrahydrate, isomalt, sorbitol, citric acid, sodium citrate, methyl paraben, propyl paraben, and cherry flavor.

Preferably said single-dose stick pack contains about 10 ml of an aqueous suspension comprising calcium citrate tetrahydrate in an amount equivalent to a unitary dose of about 1000 mg of calcium, isomalt, and sorbitol.

Still more preferably, said single-dose stick pack containing about 10 ml of the aqueous suspension of the invention, contains calcium citrate tetrahydrate in an amount equivalent to a unitary dose of about 1000 mg of calcium, about 0.5 g of isomalt, about 4 g of sorbitol, about 30 mg of citric acid, about 15 mg of sodium citrate, about 15 mg of methyl paraben, about 3 mg of propyl paraben, and about 10 mg of cherry flavor.

Therefore, in such embodiments of the invention, it is possible to obtain suspensions comprising calcium citrate wherein calcium is in a concentration of up to about 100 mg in 1 ml of suspension.

In a further embodiment of the invention, said single-dose stick pack contains about 10 ml of an aqueous suspension comprising calcium citrate tetrahydrate in an amount equivalent to a unitary dose of about 500 mg of calcium, about 500 mg of isomalt, about 2.5 g of sorbitol, about 15 mg of citric acid, about 8 mg of sodium citrate, about 8 mg of methyl paraben, about 2 mg of propyl paraben, and about 5 mg of cherry flavor.

As it will also be apparent from the experimental part that follows, the formulations in the form of an aqueous suspension of the invention, comprising isomalt in combination with calcium citrate, proved to be particularly stable even when using high doses of calcium citrate and small suspension volumes.

The formulations of the invention resulted therefore advantageous to be easily taken by any type of patient in need of calcium supplementation, in particular elderly patients and pediatric patients.

The compositions of the invention further comprising sorbitol exhibit marked performances both under the rheological and organoleptic profile.

It is to be understood that all the aspects identified as preferred and advantageous for the aqueous suspension formulations comprising calcium citrate described above are to be considered likewise preferred and advantageous also for the single-dose containers containing said formulations, and respective uses thereof in the treatment of patients in need of calcium supplementation.

Examples of embodiments of the present invention are provided below as non-limiting examples.

### Experimental Part

### Example 1

With the aim of obtaining stable suspensions, various solutions were prepared by dispersing calcium citrate tetrahydrate in deionized water in the presence of various excipients having solubilizing, complexing, cosolving, stabilizing, and viscosizing properties.

In the following Tables 1a and 1b, some exemplary formulations among the dozens of tested formulations are reported, in particular A-N formulations obtained by mixing in deionized water the various ingredients in the weight proportions shown in the table are reported.

**Table 1a**

| | **A** | **B** | **C** | **D** | **E** | **F** |
|---|---|---|---|---|---|---|
| **Ingredients** | **grams** | **grams** | **grams** | **grams** | **grams** | **grams** |
| deionized water | 25.000 | 10.000 | 10.000 | 10.000 | 7.000 | 4.000 |
| calcium citrate tetrahydrate | 4.745 | 4.745 | 4.745 | 4.745 | 4.745 | 4.745 |
| tartaric acid | 4.745 | | | | | |
| malic acid | | | | 1.000 | 1.000 | |
| maleic acid | | | | 0.050 | | |
| citric acid | | | | | 2.000 | |
| 80% lactic acid | | | | | | 0.200 |
| sodium carboxymethylcellulose | 0.500 | | | | | |
| PEG200 | | | | | | 4.000 |
| PG alginate | | | | | | 0.200 |
| sodium citrate | | 1.000 | 1.000 | 1.000 | 1.500 | |
| sodium gluconate | | | 0.500 | | 2.000 | |
| mannitol | | 0.200 | 0.200 | 0.200 | | |
| sorbitol | | | | | 0.250 | |
| sodium cyclamate | | | | | 0.200 | |
| ***total*** | ***34.990*** | ***15.945*** | ***16.445*** | ***16.995*** | ***18.695*** | ***13.145*** |
| **Suspension appearance** | | | | | | |
| At time point t=0 | fluid | fluid | fluid | fluid | fluid | fluid |
| At time point t=24 hours | fluid | cake | very viscous | almost-cake | cake | very viscous |
| At time point t=48 hours | very viscous | / | cake | cake | / | cake |

**Table 1b**

| | **G** | **H** | **I** | **L** | **M** | **N** |
|---|---|---|---|---|---|---|
| **Ingredients** | **grams** | **grams** | **grams** | **grams** | **grams** | **grams** |
| deionized water | 10.000 | 8.000 | 10.000 | 9.000 | 4.000 | 4.000 |
| calcium citrate tetrahydrate | 4.745 | 4.745 | 4.745 | 4.745 | 4.745 | 4.745 |
| malic acid | 1.000 | | 4.000 | 1.000 | 1.000 | 1.000 |
| maleic acid | 0.050 | 0.050 | | | | |
| citric acid | | 1.000 | | | | |
| sodium carboxymethylcellulose | | 0.400 | | | | |
| polyvinylpyrrolidone | 0.400 | | | | | |
| glycerin | | | | | 4.000 | 4.000 |
| sodium citrate | 1.000 | 1.000 | | | | |
| sodium gluconate | 2.000 | | 2.000 | 1.000 | 1.000 | 1.000 |
| sodium glucono-delta-lactone | | 1.000 | 2.000 | 1.000 | 1.000 | 1.000 |
| mannitol | | | 0.150 | | | |
| sorbitol | | | | 0.200 | | |
| isomalt | | | | 0.100 | 0.100 | 0.400 |
| sodium saccharinate | | | | 0.500 | 0.500 | 0.500 |
| ***total*** | ***19.195*** | ***16.195*** | ***22.895*** | ***17.545*** | ***16.345*** | ***16.645*** |
| **Suspension appearance** | | | | | | |
| At time point t=0 | fluid | fluid | fluid | fluid | fluid | fluid |
| At time point t=24 hours | very viscous | very viscous | very viscous | fluid with partial sedimentation (easily redispersible sediment) | fluid with partial sedimentation (easily redispersible sediment) | fluid with partial sedimentation (easily redispersible sediment) |
| At time point t=48 hours | very viscous | cake | cake | fluid with partial sedimentation (easily redispersible sediment) | fluid with partial sedimentation (easily redispersible sediment) | fluid with partial sedimentation (easily redispersible sediment) |

Once the various aqueous suspensions were prepared, their observation over time was carried out to verify their stability, and therefore the potential occurrence of sedimentation phenomena.

As reported in the same Tables 1a and 1b, in correspondence of each formulation, the appearance and the behavior of the suspension was recorded at the time of preparation (time point t=0), after 24 hours from preparation (time point t=24 hours), and finally after 48 hours from preparation (time point t=48 hours).

A-I suspensions proved to be completely unsatisfactory, due to more or less rapid sediment formation phenomena (cake) no longer redispersible, or excessive viscosity.

Only L-N formulations, comprising isomalt allowed to obtain suspensions that remained fluid even after 48 hours from preparation thereof.

Over an observation period of about 20 days in total, it was observed that these suspensions showed a tendency to form sediments which, however, were easily redispersible, thus making their use as formulations for oral administration completely reasonable.

### Example 2

In light of the excellent results obtained in the case of L-N formulations of Example 1, a series of optimization tests were carried out to obtain increasingly stable suspensions, while at the same time trying to define the best sweeteners to be added in order to obtain good palatability, as well as the most suitable flavoring agents to mask the acidity.

The additional O-X formulations described in Table 2 were then prepared.

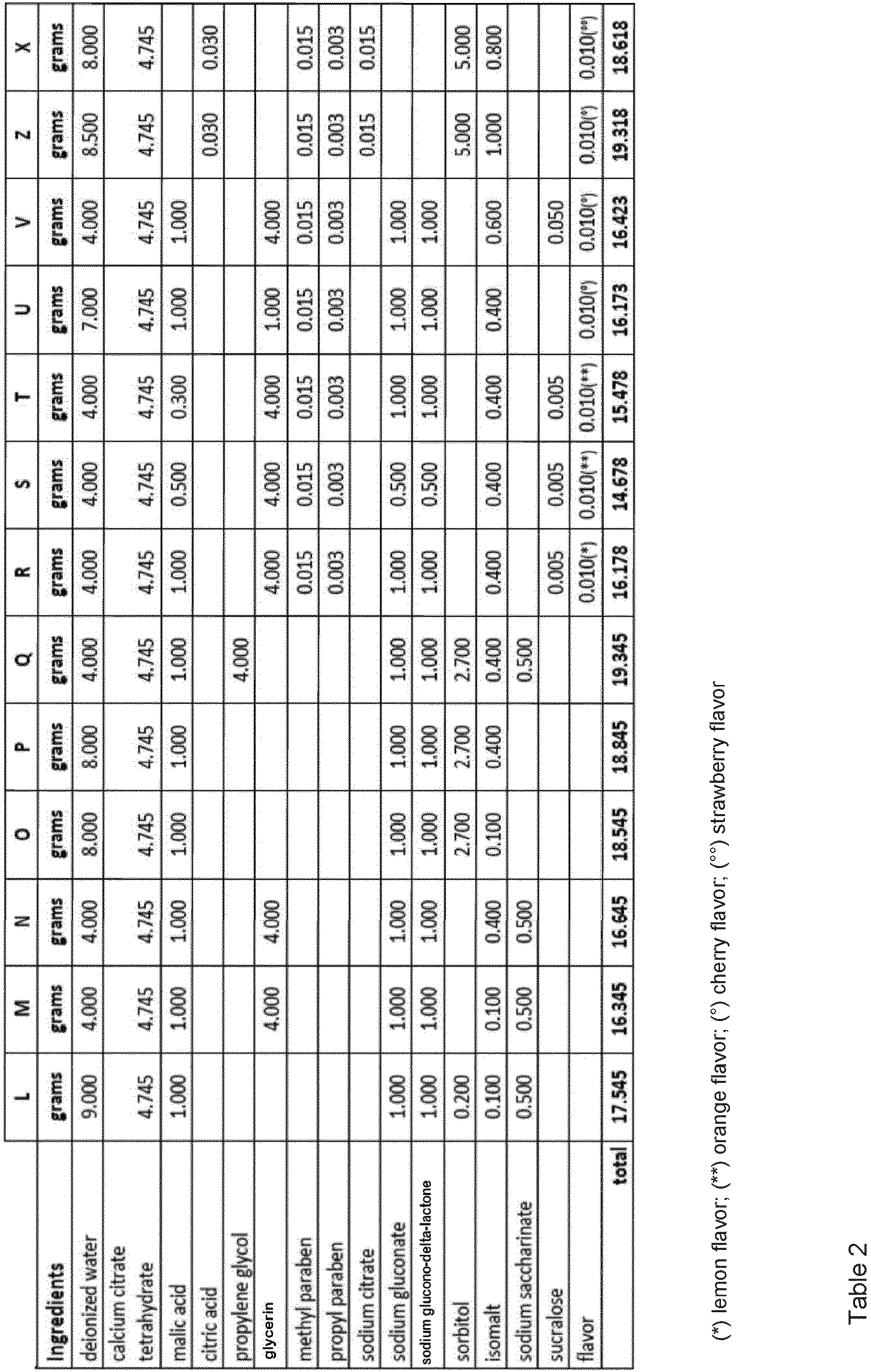

As it is apparent from the data shown in Table 2, many different excipients were varied and tested, in particular organic acids, solubilizers, sweeteners, cosolvents, always maintaining the presence of isomalt, which, based on the results of the tests carried out in the test described in Example 1, was surprisingly found to be an essential ingredient to ensure obtainment of stable suspensions.

The evidence that the presence of isomalt in suspensions comprising calcium citrate was a fundamental element to ensure their stability was also confirmed by the present test.

All the additional O-X suspensions prepared, characterized by very different qualitative compositions, proved to be in any case stable, with an index of redispersibility of the sediment possibly formed from good to excellent, thanks to the presence of isomalt.

In addition, the performance of the suspensions containing sorbitol in combination with isomalt appeared in general superior, both in terms of stability and organoleptic characteristics.

Among these, in particular, the formulation Z proved to be the best in every respect.

### Example 3

Considering that the presence in the calcium citrate suspension of the isomalt and sorbitol ingredients conferred the best physical and organoleptic characteristics to the preparation, further formulations were prepared, in particular by modifying the amounts of the ingredients in formulation Z, *i.e.* the best formulation that had been obtained in the previous tests, described in Example 2.

In particular, the influence of isomalt and sorbitol concentration on stability and viscosity was thus checked, since this parameter is one of the most important to consider in the production of marketable formulations in stick pack format, as well as palatability and pleasantness.

Z1-Z4 formulations described in the following Table 3 were thus prepared (amounts expressed in grams).

**Table 3.**

| | **Z** | **Z1** | **Z2** | **Z3** | **Z4** |
|---|---|---|---|---|---|
| water | 8.500 | 8.500 | 8.500 | 8.500 | 8.500 |
| calcium citrate | 4.745 | 4.745 | 4.745 | 4.745 | 4.745 |
| isomalt | 1.000 | 0.500 | 0.100 | 1.000 | 0.500 |
| sorbitol | 5.000 | 5.000 | 5.000 | 3.000 | 3.000 |
| citric acid | 0.030 | 0.030 | 0.030 | 0.030 | 0.030 |
| sodium citrate | 0.015 | 0.015 | 0.015 | 0.050 | 0.050 |
| methyl paraben | 0.015 | 0.015 | 0.015 | 0.015 | 0.015 |
| propyl paraben | 0.003 | 0.003 | 0.003 | 0.003 | 0.003 |
| flavor | 0.010 | 0.010 | 0.010 | 0.010 | 0.010 |
| total | **19.318** | **18.818** | **18.418** | **17.353** | **16.853** |
| | | | | | |
| *viscosity (Pa·s)* | *4.307* | *4.911* | *5.801* | *6.857* | *1.500* |

All formulations proved to be stable, with completely acceptable viscosity values, adequate rheology, pleasing in terms of organoleptic properties, and adequate to be packaged in the stick pack format.

In particular, the test results also showed that it was possible to reduce the amounts of both isomalt and sorbitol, while continuing to obtain suspensions with characteristics suitable for use.

Specifically, it was possible to obtain stable and pleasant suspensions even with isomalt amounts lower than 1000 mg per unitary dose, *i.e.* in the range from 100 to 1000 mg, and with sorbitol amounts lower than 5 g per unitary dose, *i.e.*in the range from 3 to 5 g.

The possibility of reducing the amounts of excipients is obviously an aspect of interest, both from an industrial point of view, considering that this would entail lower costs of production, and from a pharmaceutical compliance point of view, considering that this would allow the preparation of pharmaceutical preparations or nutritional supplements in stick pack formats characterized by lower suspension volumes, and thus easier to take by any type of patient, especially children and the elderly.

### Example 4

In order to further reduce the preparation volume, tests to reduce the amount of water were also carried out.

In particular, the Z5 formulation was prepared, as a further variant of the Z formulation, whose qualitative and quantitative composition is reported in the following Table 4 (amounts expressed in grams).

**Table 4.**

| | **Z** | **Z5** |
|---|---|---|
| water | 8.500 | 4.682 |
| calcium citrate | 4.745 | 4.745 |
| isomalt | 1.000 | 0.500 |
| sorbitol | 5.000 | 4.000 |
| citric acid | 0.030 | 0.030 |
| sodium citrate | 0.015 | 0.015 |
| methyl paraben | 0.015 | 0.015 |
| propyl paraben | 0.003 | 0.003 |
| flavor | 0.010 | 0.010 |
| **Total amount in grams** | **19.318** | **14.000** |
| density (g/cm³) | 1.424 | 1.371 |
| **Volume (ml)** | **13.566** | **10.211** |

The experiment showed that, despite the considerable reduction in the amount of water, with the achievement of a total volume for unitary dose preparation of just over 10 ml, it was still possible to obtain a completely stable suspension with good organoleptic characteristics.

The solubilizing/stabilizing synergistic effect of the combination of calcium citrate with isomalt and sorbitol made it possible to prepare stable suspensions with a very high content of calcium citrate, capable of providing an equivalent amount of calcium equal to about 100 mg/ml of suspension.

Given the small total volume of the preparation, the Z5 formulation, which can be realized in the stick pack format, clearly proved to be of considerable interest from a commercial point of view, thanks to its superior pharmaceutical compliance.

### Example 5

A further Z6 formulation, described in the following Table 5, was prepared with the aim of obtaining a stick pack formulation in the unitary dosage of 500 mg of calcium.

**Table 5**

| **Ingredients** | **g** |
|---|---|
| deionized water | 3.300 |
| calcium citrate tetrahydrate | 2.372 |
| isomalt | 0.500 |
| sorbitol (70% solution) | 3.570 |
| citric acid | 0.015 |
| sodium citrate | 0.008 |
| methyl paraben | 0.008 |
| propyl paraben | 0.002 |
| cherry flavor | 0.005 |
| ***total*** | ***9.780*** |
| *viscosity (Pa·s)* | *4.771* |

Also this formulation proved to be stable and pleasing to taste, thus confirming the possibility of making pharmaceutical preparations and nutritional supplements based on calcium citrate in any unitary dosage, in particular also the 500 mg calcium dosage.

In conclusion, the formulations in the form of an aqueous suspension of the invention, comprising isomalt in combination with calcium citrate, proved to be surprisingly stable, even after long observation times, also when using high doses of calcium citrate in small suspension volumes.

In particular, the calcium citrate suspensions comprising also sorbitol proved to be particularly performing, with high stability and excellent organoleptic characteristics. The formulations of the invention are also particularly advantageous as they allow the administration in a single dose of the entire recommended daily dose of calcium, that is 1000 mg of calcium, *inter alia* readily bioavailable, being in the form of calcium citrate.

The formulations of the invention nevertheless allow the preparation of each type of unitary dose, such as for example the unitary dose of 500 mg of calcium.

The aqueous suspension formulations of calcium citrate comprising hence isomalt and, preferably, also sorbitol, therefore represent an ideal solution for the administration of calcium in a readily bioavailable form, such as calcium citrate, to patients who are in need of supplementation thereof to prevent or counteract all the diseases related to bone mass loss, such as osteoporosis and fractures.

## Claims

1. A formulation in the form of an aqueous suspension comprising calcium citrate and isomalt.

2. The formulation in the form of an aqueous suspension according to claim 1, wherein calcium citrate is calcium citrate tetrahydrate.

3. The formulation in the form of an aqueous suspension according to claims 1 or 2, further comprising sorbitol.

4. The formulation in the form of an aqueous suspension according to any one of claims 1 to 3, wherein calcium citrate is in a concentration, expressed with reference to calcium, in the range from 20 to 100 mg per 1 ml of suspension, preferably in the range from 40 to 70 mg per 1 ml of suspension, more preferably of about 50 mg per 1 ml of suspension.

5. The formulation in the form of an aqueous suspension according to any one of claims 1 to 4, wherein isomalt is in a percentage concentration (w/w) in the range from 0.5 to 10%(w/w), preferably from 2 to 6%(w/w) with respect to the weight of the final formulation.

6. The formulation in the form of an aqueous suspension according to any one of claims 1 to 5, wherein sorbitol is in a percentage concentration (w/w) in the range from 10 to 30%(w/w), preferably from 17 to 28%(w/w) with respect to the weight of the final formulation.

7. The formulation in the form of an aqueous suspension according to any one of claims 1 to 6, wherein the aqueous suspension is in the form of a unitary dose.

8. The formulation in the form of an aqueous suspension according to claim 7, wherein the aqueous suspension in the form of a unitary dose comprises calcium citrate in an amount corresponding to a unitary dose of calcium in the range from 200 to 1500 mg, preferably from 400 to 1200 mg, still more preferably of about 500 mg or about 1000 mg.

9. The formulation in the form of an aqueous suspension according to any one of claims 7 to 8, wherein isomalt is in an amount from 0.1 to 1 g, preferably from 0.4 to 1 g.

10. The formulation in the form of an aqueous suspension according to any one of claims 7 to 9, wherein sorbitol is in an amount in the range from 1 to 5 g, preferably from 2.5 to 5 g.

11. The formulation in the form of an aqueous suspension according to any one of claims 7 to 10, wherein the total volume of said aqueous suspension is in the range from 5 to 30 ml, preferably from 8 to 25 ml, more preferably from 9 to 15 ml, still more preferably is of about 10 ml.

12. The formulation in the form of an aqueous suspension according to any one of claims 7 to 11 comprising calcium citrate in an amount corresponding to a unitary dose of calcium of 1000 mg in a total volume of about 10 ml.

13. The formulation in the form of an aqueous suspension according to any one of claims 7 to 12 wherein the total weight of said aqueous suspension is in the range from 5 to 20 g, preferably comprised in the range from 6 to 10 g for the unitary dose delivering an equivalent amount of calcium of about 500 mg, and in the range from 16 to 20 g for the unitary dose delivering an equivalent amount of calcium of about 1000 mg.

14. The formulation in the form of an aqueous suspension according to claim 1, further comprising citric acid and sodium citrate.

15. A single-dose flexible container containing a unitary dose of a pharmaceutical preparation or nutritional supplement comprising a formulation according to any one of claims 1 to 14.

16. The container according to claim 15, wherein the flexible container is a cheer pack or a stick pack, preferably a stick pack.

17. A formulation in the form of an aqueous suspension according to any one of claims 1 to 14, or a container according to any one of claims 15 to 16, for use in the treatment of subjects in need of calcium supplementation.

18. The formulation for use or the container for use according to claim 17, wherein said subjects in need of calcium supplementation are elderly patients or pediatric patients.

19. The formulation for use or the container for use according to claim 17, wherein said subjects in need of calcium supplementation are patients affected by diseases related to bone mass loss, such as diseases selected from the group consisting of osteoporosis, fractures, chronic diarrhea syndromes, hypertension, colon cancer.

## Patentansprüche

1. Rezeptur in der Form einer wässrigen Suspension, die Calciumcitrat und Isomalt umfasst.

2. Rezeptur in der Form einer wässrigen Suspension gemäß Anspruch 1, wobei das Calciumcitrat Calciumcitrat-Tetrahydrat ist.

3. Rezeptur in der Form einer wässrigen Suspension gemäß Anspruch 1 oder 2, die ferner Sorbitol umfasst.

4. Rezeptur in der Form einer wässrigen Suspension gemäß einem der Ansprüche 1 bis 3, wobei Calciumcitrat in einer Konzentration enthalten ist, die, bezogen auf Calcium ausgedrückt, im Bereich von 20 bis 200 mg pro 1 ml Suspension, vorzugsweise im Bereich von 40 bis 70 mg pro 1 ml Suspension, noch bevorzugter ungefähr bei 50 mg pro 1 ml Suspension liegt.

5. Rezeptur in der Form einer wässrigen Suspension gemäß einem der Ansprüche 1 bis 4, wobei Isomalt in einem Massenanteil im Bereich von 0,5 bis 10 Gew.-%, vorzugsweise von 2 bis 6 Gew.-% bezogen auf das Gewicht der letztendlichen Rezeptur enthalten ist.

6. Rezeptur in der Form einer wässrigen Suspension gemäß einem der Ansprüche 1 bis 5, wobei Sorbitol in einem Massenanteil im Bereich von 10 bis 30 Gew.-%, vorzugsweise von 17 bis 28 Gew.-% bezogen auf das Gewicht der letztendlichen Rezeptur enthalten ist.

7. Rezeptur in der Form einer wässrigen Suspension gemäß einem der Ansprüche 1 bis 6, wobei die wässrige Suspension in der Form einer Einheitsdosis ist.

8. Rezeptur in der Form einer wässrigen Suspension gemäß Anspruch 7, wobei die wässrige Suspension in der Form einer Einheitsdosis Calciumcitrat in einer Menge umfasst, die einer Einheitsdosis von Calcium im Bereich von 200 bis 1500 mg, vorzugsweise von 400 bis 1200 mg, noch bevorzugter von ungefähr 500 mg oder ungefähr 1000 mg entspricht.

9. Rezeptur in der Form einer wässrigen Suspension gemäß einem der Ansprüche 7 bis 8, wobei Isomalt in einer Menge von 0,1 bis 1 g, vorzugsweise von 0,4 bis 1 g enthalten ist.

10. Rezeptur in der Form einer wässrigen Suspension gemäß einem der Ansprüche 7 bis 9, wobei Sorbitol in einer Menge im Bereich von 1 bis 5 g, vorzugsweise von 2,5 bis 5 g enthalten ist.

11. Rezeptur in der Form einer wässrigen Suspension gemäß einem der Ansprüche 7 bis 10, wobei das Gesamtvolumen der wässrigen Suspension im Bereich von 5 bis 30 ml, vorzugsweise von 8 bis 25 ml, noch bevorzugter von 9 bis 15 ml, noch bevorzugter ungefähr 10 ml ist.

12. Rezeptur in der Form einer wässrigen Suspension gemäß einem der Ansprüche 7 bis 11, umfassend Calciumcitrat in einer Menge, die einer Einheitsdosis von Calcium von 1000 mg in einem Gesamtvolumen von ungefähr 10 ml entspricht.

13. Rezeptur in der Form einer wässrigen Suspension gemäß einem der Ansprüche 7 bis 12, wobei das Gesamtgewicht der wässrigen Suspension im Bereich von 5 bis 20 g, vorzugsweise im Bereich von 6 bis 10 g für die Einheitsdosis liegt, die eine äquivalente Menge von Calcium von ungefähr 500 mg liefert, und im Bereich von 16 bis 20 g für die Einheitsdosis liegt, die eine äquivalente Menge von Calcium von ungefähr 1000 mg liefert.

14. Rezeptur in der Form einer wässrigen Suspension gemäß Anspruch 1, ferner umfassend Citronensäure und Natriumcitrat.

15. Flexibler Einzeldosisbehälter, der eine Einheitsdosis einer pharmazeutischen Zubereitung oder eines Nahrungsergänzungsmittels enthält, die bzw. das eine Rezeptur gemäß einem der Ansprüche 1 bis 14 umfasst.

16. Behälter gemäß Anspruch 15, wobei der flexible Behälter ein Cheer-Pack oder Stick-Pack, vorzugsweise ein Stick-Pack ist.

17. Rezeptur in der Form einer wässrigen Suspension gemäß einem der Ansprüche 1 bis 14, oder Behälter gemäß einem der Ansprüche 15 bis 16, zur Verwendung in der Behandlung von Menschen, die Calcium als Nahrungsergänzung benötigen.

18. Rezeptur zur Verwendung oder Behälter zur Verwendung gemäß Anspruch 17, wobei die Menschen, die Calcium als Nahrungsergänzung benötigen, ältere Patienten oder pädiatrische Patienten sind.

19. Rezeptur zur Verwendung oder Behälter zur Verwendung gemäß Anspruch 17, wobei die Menschen, die Calcium als Nahrungsergänzung benötigen, Patienten sind, die an Krankheiten leiden, die mit dem Verlust an Knochenmasse einhergehen, wie zum Beispiel Krankheiten, die aus der Gruppe ausgewählt sind, die aus Osteoporose, Frakturen, chronische Diarrhö-Syndrome, Bluthochdruck, Kolonkrebs besteht.

## Revendications

1. Formulation sous forme d'une suspension aqueuse comprenant du citrate de calcium et de l'isomalt.

2. Formulation sous forme d'une suspension aqueuse selon la revendication 1, dans laquelle du citrate de calcium est du citrate de calcium tétrahydrate.

3. Formulation sous forme d'une suspension aqueuse selon les revendications 1 ou 2, comprenant en outre du sorbitol.

4. Formulation sous forme d'une suspension aqueuse selon l'une quelconque des revendications 1 à 3, dans laquelle du citrate de calcium est dans une concentration, exprimées par rapport au calcium, dans la plage de 20 à 100 mg pour 1 ml de suspension, préférentiellement dans la plage de 40 à 70 mg pour 1 ml de suspension, plus préférentiellement d'environ 50 mg pour 1 ml de suspension.

5. Formulation sous forme d'une suspension aqueuse selon l'une quelconque des revendications 1 à 4, dans laquelle de l'isomalt est dans une concentration en pourcentage (p/p) dans la plage de 0,5 à 10 % (p/p), préférentiellement de 2 à 6 % (p/p) par rapport au poids de la formulation finale.

6. Formulation sous forme d'une suspension aqueuse selon l'une quelconque des revendications 1 à 5, dans laquelle du sorbitol est dans une concentration en pourcentage (p/p) dans la plage de 10 à 30 % (p/p), préférentiellement de 17 à 28 % (p/p) par rapport au poids de la formulation finale.

7. Formulation sous forme d'une solution aqueuse selon l'une quelconque des revendications 1 à 6, dans laquelle la suspension aqueuse est sous forme d'une dose unitaire.

8. Formulation sous forme d'une suspension aqueuse selon la revendication 7, dans laquelle la suspension aqueuse sous forme d'une dose unitaire comprend du citrate de calcium dans une quantité correspondant à une dose unitaire de calcium dans la plage de 200 à 1500 mg, préférentiellement de 400 à 1200 mg, encore plus préférentiellement d'environ 500 mg ou environ 1000 mg.

9. Formulation sous forme d'une suspension aqueuse selon l'une quelconque des revendications 7 à 8, dans laquelle de l'isomalt est dans une quantité de 0,1 à 1 g, préférentiellement de 0,4 à 1 g.

10. Formulation sous forme d'une suspension aqueuse selon l'une quelconque des revendications 7 à 9, dans laquelle du sorbitol est dans une quantité dans la plage de 1 à 5 g, préférentiellement de 2,5 à 5 g.

11. Formulation sous forme d'une suspension aqueuse selon l'une quelconque des revendications 7 à 10, dans laquelle le volume total de ladite suspension aqueuse est dans la plage de 5 à 30 ml, préférentiellement de 8 à 25 ml, plus préférentiellement de 9 à 15 ml, encore plus préférentiellement est d'environ 10 ml.

12. Formulation sous forme d'une suspension aqueuse selon l'une quelconque des revendications 7 à 11 comprenant du citrate de calcium dans une quantité correspondant à une dose unitaire de calcium de 1000 mg dans un volume total d'environ 10 ml.

13. Formulation sous forme d'une suspension selon l'une quelconque des revendications 7 à 12, dans laquelle le poids total de ladite suspension aqueuse est dans la plage de 5 à 20 g, préférentiellement compris dans la plage de 6 à 10 g pour la dose unitaire apportant une quantité équivalente de calcium d'environ 500 mg, et dans la plage de 16 à 20 g pour la dose unitaire apportant une quantité équivalente de calcium d'environ 1000 mg.

14. Formulation sous forme d'une suspension aqueuse selon la revendication 1, comprenant en outre de l'acide citrique et du citrate de sodium.

15. Conteneur flexible à dose unique contenant une dose unitaire d'une préparation pharmaceutique ou d'un complément nutritionnel comprenant une formulation selon l'une quelconque des revendications 1 à 14.

16. Conteneur selon la revendication 15, dans lequel le conteneur flexible est un emballage cheer pack ou un emballage en bâtonnets, préférentiellement un emballage en bâtonnets.

17. Formulation sous forme d'une suspension aqueuse selon l'une quelconque des revendications 1 à 14, ou conteneur selon l'une quelconque des revendications 15 à 16, pour une utilisation dans le traitement de sujets ayant besoin d'une supplémentation en calcium.

18. Formulation pour une utilisation ou conteneur pour une utilisation selon la revendication 17, dans lesquels lesdits sujets ayant besoin d'une supplémentation en calcium sont des patients âgés ou des patients pédiatriques.

19. Formulation pour une utilisation ou conteneur pour une utilisation selon la revendication 17, dans lesquels lesdits sujets ayant besoin d'une supplémentation en calcium sont des patients souffrant de maladies liées à la perte de masse osseuse, telles que des maladies sélectionnées parmi le groupe constitué d'ostéoporose, fractures, syndromes de diarrhée chronique, hypertension, cancer du côlon.
